# EUROPEAN PATENT APPLICATION

(11) **EP 4 368 245 A1**
(43) Date of publication of application: **15.05.2024**
(21) Application number: 22837775.0
(22) Date of filing: 05.01.2022
(51) Int. Cl.: A61N 1/40, A61N 1/08, A61N 1/06, A61N 1/32, A61B 5/0531

(54) **REAL-TIME IMPEDANCE MATCHING METHOD FOR HIGH FREQUENCY TREATMENT DEVICE**

(30) Priority: 09.07.2021 KR 20210090447
(71) Applicant: Tentech Inc., Seoul 06103 (KR)
(72) Inventor: KIM, Ki Hang, Seoul 04799 (KR)
(74) Representative: Gille Hrabal Partnerschaftsgesellschaft mbB Patentanwälte
(86) International application number: PCT/KR2022/000186
(87) International publication number: WO 2023/282420

(57) **Abstract**

Provided is a real-time impedance matching method for a high-frequency treatment device, the method including: fixing, to a preset inductance in a reference impedance setting step, inductance of a first variable inductor part for measuring impedance in the high-frequency treatment device and inductance of a second variable inductor part for measuring impedance in a patient's body; applying, in a minimum impedance search step, a search voltage lower than a medical procedure voltage and searching for a minimum impedance by changing the inductance of the first variable inductor part and the inductance of the second variable inductor part; measuring, in a reflected wave measurement step, at least two reflected waves under a condition in which the inductance of the first variable inductor part and the inductance of the second variable inductor part are varied; comparing, in a reflected wave minimum value search step, the measured reflected waves to search for the reflected wave having a minimum value; determining, in an inductance determination step, the inductance of the first variable inductor part and the inductance of the second variable inductor part that satisfy the reflected wave minimum value; and operating, in a high-frequency application step, the high-frequency treatment device with the medical procedure voltage to apply high-frequency waves to the patient's skin under a condition of the inductance of the first variable inductor part and the inductance of the second variable inductor part determined in the inductance determination step.

## Description

### Technical Field

The present disclosure relates to a real-time impedance matching method for a high-frequency treatment device. More particularly, the present disclosure relates to a real-time impedance matching method for a high-frequency treatment device, the method being capable of matching impedance in the high-frequency treatment device to impedance in a patient's body during high-frequency treatment.

### Background Art

Human skin tissue consists of the stratum corneum, epidermis, dermis, and hypodermis, and functions of the tissue deteriorate due to aging and effects from ultraviolet rays.

The epidermis is the outermost layer of the skin and is composed of several layers such as the stratum corneum, stratum lucidum, stratum granulosum, tratum spinosum, and basal layer depending on positions and functions, and is responsible for functions such as protection, defense, and secretion.

The dermis is located below the epidermis and adjacent to the basal layer, making up most of the skin. The dermis is divided into two layers: the papillary dermis and the reticular dermis. The papillary dermis is composed of water, proteins, saccharides, mucopolysaccharides, minerals, and inorganic salts in gel-like state, and has capillary vessels associated with blood circulation and lymphatic vessels carrying lymph. The reticular dermis is composed of collagen which is collagenous fiber associated with the wrinkles of the skin, elastin which is resilient fiber that provides elasticity to the skin, and a ground substrate (reservoir of water).

The hypodermis is located between the dermis, muscles, and bones, contains a large amount of fat, and forms the lowest layer of the skin. The hypodermis spreads evenly throughout the human body, providing elasticity maintenance and a buffering action of absorbing external pressure and impact, thereby not only protecting the inside of the body against damages, but also preventing the loss of body heat and maintaining body temperature.

In the meantime, there has been a growing interest in skin, so various skin care devices related to skin care have been developed. Simultaneously, skin specialty hospitals and skin care medical procedure companies have been founded.

Recently, the most widely known skin medical procedure method is as follows: radio frequency (RF) waves are applied to the skin to cause local thermal damage in the skin, making tiny wounds for facilitating regeneration of the skin ranging from the epithelium to the dermis, and the tiny wounds induce cell growth factors to maximize natural healing and regeneration of the skin.

A high-frequency treatment device is mainly used to heat subcutaneous fat with electromagnetic energy using radio frequency (RF) waves to irreversibly denature the subcutaneous fat and cause fat reduction through apoptosis.

However, the impedance of the skin varies among individuals, and varies depending on the location and condition of the skin even for the same person. Accordingly, when the impedance changes, the high-frequency power delivered to the skin also changes proportionally, making it difficult to expect the high-frequency treatment device to achieve consistent power delivery. In addition, in the skin with low impedance, the current transferred to the skin increases and the heat generated in the deep area increase quickly, making burns easily. Conversely, in the region with high impedance, it takes a long time to generate heat, making it practically impossible to achieve uniform heating.

Furthermore, when there is a difference between the impedance in the device and the impedance in a patient's body, energy transfer efficiency is reduced and high-frequency output is unstable.

### Disclosure

### Technical Problem

The present disclosure has been made keeping in mind the above problems occurring in the related art, and the present disclosure is directed to providing a real-time impedance matching method for a high-frequency treatment device, wherein impedance in the high-frequency treatment device is matched to impedance in a patient's body so that energy is efficiently provided to the patient's skin during high-frequency treatment and the output of high-frequency waves is maintained stably.

### Technical Solution

The objective of the present disclosure can be achieved by a real-time impedance matching method for a high-frequency treatment device, the method including: fixing, to a preset inductance in a reference impedance setting step, inductance of an impedance matching part for measuring impedance in the high-frequency treatment device and impedance in a patient's body; applying, in a minimum impedance search step, a search voltage lower than a medical procedure voltage and searching for a minimum impedance by changing the inductance of the impedance matching part; measuring, in a reflected wave measurement step, at least two reflected waves under a condition in which the inductance of the impedance matching part is varied; comparing, in a reflected wave minimum value search step, the measured reflected waves to search for a reflected wave minimum value; determining, in an inductance determination step, the inductance of the impedance matching part that satisfies the reflected wave minimum value; and operating, in a high-frequency application step, the high-frequency treatment device with the medical procedure voltage to apply high-frequency waves to the patient's skin under a condition of the inductance of the impedance matching part determined in the inductance determination step.

According to the real-time impedance matching method for the high-frequency treatment device according to an embodiment of the present disclosure, after the high-frequency application step, the steps starting from the reflected wave measurement step may be repeated when it is intended to apply high-frequency waves to a new medical procedure position on the patient's skin.

According to an embodiment of the present disclosure, in the reflected wave minimum value search step, while the inductance of the impedance matching part is varied, a plurality of inflection points of measured reflected wave intensity may be found, and the reflected wave minimum value having a lowest reflected wave intensity among the plurality of inflection points may be found.

According to the real-time impedance matching method for the high-frequency treatment device according to an embodiment of the present disclosure, when it is intended to search for a reflected wave minimum value at the new medical procedure position, reflected waves at the new medical procedure position may be found preferentially using inductance corresponding to a reflected wave minimum value at a previous medical procedure position and an inductance value adjacent to the reflected wave minimum value.

According to an embodiment of the present disclosure, the impedance matching part may include: a first variable inductor part configured to measure the impedance in the high-frequency treatment device; and a second variable inductor part configured to measure the impedance in the patient's body,

wherein inductance of the first variable inductor part and inductance of the second variable inductor part may be the same.

In addition, each of the first variable inductor part and the first variable inductor part may include at least two unit inductors connected in series, and each of the at least two unit inductors may include an inductor and a switch connected in parallel.

In addition, a plurality of the inductors constituting the unit inductors may have different proportionality constants.

### Advantageous Effects

According to the present disclosure, the real-time impedance matching method for the high-frequency treatment device matches the impedance in the high-frequency treatment device to the impedance in a patient's body to efficiently transfer energy to the skin when high-frequency waves are applied, thereby minimizing power usage and performing treatment efficiently.

Furthermore, the temperature of subcutaneous fat can be heated consistently and uniformly, so that fat dissolving efficiency can be improved and burns caused by temperature changes can be prevented, thus improving safety during treatment.

### Description of Drawings

FIG. 1 is a block diagram illustrating an impedance matching device for a high-frequency treatment device according to the present disclosure.
FIG. 2 is a circuit diagram illustrating an equivalent circuit of an impedance matching device for a high-frequency treatment device according to an embodiment of the present disclosure.
FIG. 3 is a flowchart illustrating an example of an impedance matching method for a high-frequency treatment device according to the present disclosure.
FIG. 4 is a conceptual diagram illustrating a method of matching impedance while changing a medical procedure region according to an embodiment of the present disclosure.

### Best Mode

Similar reference numbers in the drawings refer to identical or similar functions across various aspects.

Hereinafter, specific details for practicing the present disclosure will be described based on embodiments with reference to the drawings. These embodiments are described in sufficient detail to enable those skilled in the art to practice the present disclosure.

It should be understood that various embodiments of the present disclosure are different from each other but are not necessarily mutually exclusive. For example, particular shapes, structures, and characteristics described herein with respect to one embodiment may be implemented in other embodiments without departing from the spirit and scope of the present disclosure.

Therefore, the following detailed description is not to be taken in a limiting sense, and the scope of the present disclosure is defined only by the appended claims, together with all equivalents of the claims if properly described.

A high-frequency treatment device according to the present disclosure includes: a handpiece for touching a patient's skin or being positioned spaced apart from the skin to emit high-frequency waves to a treatment target region of the patient; and a main body connected to the handpiece to control the operation of the handpiece. The main body includes a display means and a control means for controlling the operation of the handpiece and enableing the operation state to be checked. The handpiece is connected to the main body through a soft flexible cable.

The handpiece includes radio frequency (RF) electrodes for generating high-frequency waves. The RF electrodes generate radio frequency (RF) waves on the skin of a patient. In an embodiment of the present disclosure, the RF electrodes are limited to a mono polar type, and are composed of a main electrode and a ground electrode (plate). The main electrode is adjacent to or in contact with a medical procedure region. The ground electrode is located adjacent to the main electrode but spaced a predetermined distance apart therefrom. Between the two electrodes, an electric field is formed.

Between the two RF electrodes (the main electrode and the ground electrode), a treatment target region of a patient is positioned, and high-frequency waves are generated with the minimum power, which is less than power used in actual treatment and enables impedance matching operation, thereby performing impedance matching operation.

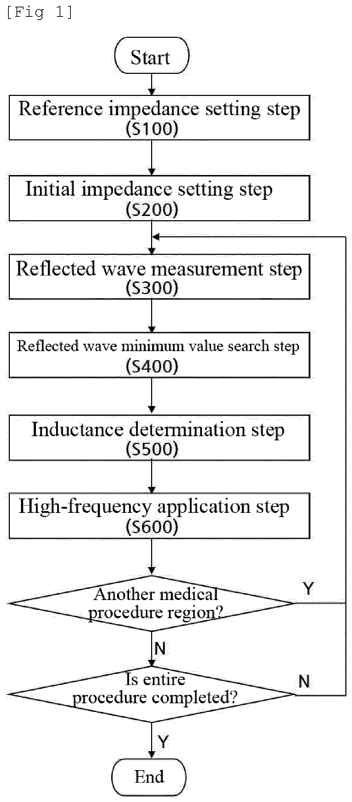

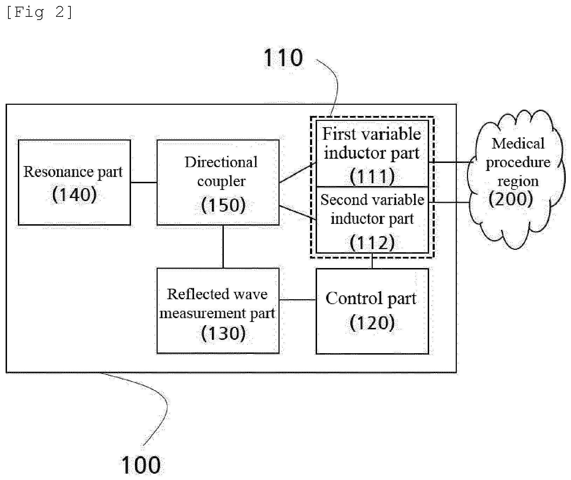

An impedance matching device 100 according to an embodiment of the present disclosure shown in FIGS. 1 and 2 is a predetermined RF circuit, and is installed in the main body or the handpiece to match the impedance of the RF electrodes of the handpiece to a patient's impedance. In this embodiment, a description of the impedance matching device installed in the handpiece will be made, but no limitation thereto is imposed.

The impedance matching device 100 for the high-frequency treatment device according to the present disclosure is capable of changing inductance, and is provided with an impedance matching part 110 capable of changing inductance for measuring the impedance in the high-frequency treatment device. The impedance matching part 110 includes a first variable inductor part 111 and a second variable inductor part 112.

The first variable inductor part 111 and the second variable inductor part 112 may change inductance. Specifically, the first variable inductor part 111 is for measuring the impedance in the high-frequency treatment device, and the second variable inductor part 112 is for measuring the impedance in a patient's body. Electrical connection to the first variable inductor part 111 is made through the patient's skin, and the impedance in the patient's body is measured through the second variable inductor part 112.

In addition, according to an embodiment of the present disclosure, the first variable inductor part 111 and the second variable inductor part 112 are composed of unit inductors, each of which an inductor having a predetermined inductance and a switch are connected in parallel. At least two such unit inductors are connected in series. The switches are opened and shorted under the control of the control part 120, thereby changing the total inductance of the first variable inductor part 111 and the second variable inductor part 112. However, no limitation thereto is imposed, and a variable inductor may be realized using various methods, such as a combination of an inductor capable of varying inductance and a capacitor, a combination with a variable resistor, or a change in inductance through physical control.

The inductors constituting the unit inductors have different proportionality constants to have different inductances. For example, it means that if one inductor has the inductance of 1.0 L, other inductors have inductances of 0.5 L and 0.33 L.

According to an embodiment of the present disclosure, each of the first variable inductor part 111 and the second variable inductor part 112 is composed of four unit inductors, for example. That is, each of the first variable inductor part 111 and the second variable inductor part 112 is composed of four inductors and respective switches, but no limitation thereto is imposed. It is preferable that at least two unit inductors are provided. Furthermore, it is preferable to combine inductors having different inductances for a combination of inductors capable of driving an optimal inductance to measure the impedance of a body.

Due to change in inductance of the first variable inductor part 111 and the second variable inductor part 112, the control part 120 matches the impedance in the high-frequency treatment device to the impedance in a patient's body through a reflected wave result value measured by a reflected wave measurement part 130.

The control part 120 controls the change in inductance of the first variable inductor part 111 and the second variable inductor part 112, and controls with feedback the inductance change of the first variable inductor part 111 and the second variable inductor part 112 on the basis of the intensity of the reflected waves measured by the reflected wave measurement part 130.

The reflection value measurement part 130 is a voltage standing wave ratio (VSWR) meter. A reflection value is measured by changing the inductance of the first variable inductor part 111 or the second variable inductor part 112.

A resonance part 140 is an LC resonance circuit in which an inductor having an inductance component of a particular value and a capacitor having a particular capacitance component are connected in series or parallel. In an embodiment of the present disclosure, the resonance part corresponds to an LC resonance circuit in which inductors and capacitors are connected in series.

A directional coupler 150 has an input port connected to the resonance part 140, has a main output port connected to the impedance matching part 110, and has an auxiliary output port connected to the reflected wave measurement part 130.

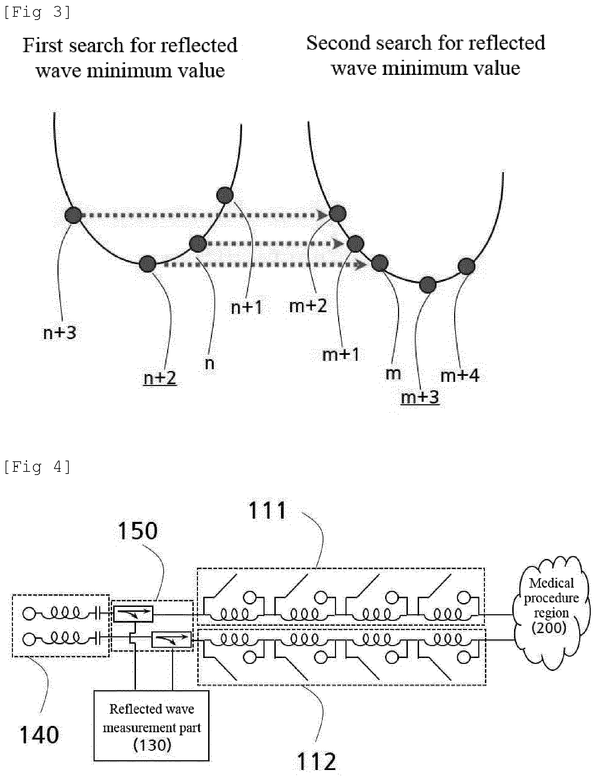

Hereinafter, a method in which the control part 120 controls the inductance of the first variable inductor part 111 and the second variable inductor part 112 to match the impedance in a patient's skin to the impedance in the high-frequency treatment device will be described in detail with reference to FIGS. 3 and 4.

A real-time impedance matching method using an impedance matching device for a high-frequency treatment device includes: fixing, to a preset inductance in a reference impedance setting step S100, inductance of an impedance matching part 110 for measuring impedance in the high-frequency treatment device 100 and impedance in a patient's body 200; applying, in a minimum impedance search step S200, a search voltage lower than a medical procedure voltage and searching for the minimum impedance by changing the inductance of the impedance matching part 110; measuring, in a reflected wave measurement step S300, at least two reflected waves under a condition in which the inductance of the impedance matching part 110 is varied; comparing, in a reflected wave minimum value search step S400, the measured reflected waves to search for a reflected wave minimum value; determining, in an inductance determination step S500, the inductance of the impedance matching part 110 that satisfies the reflected wave minimum value; and operating, in a high-frequency application step S600, the high-frequency treatment device 100 with the medical procedure voltage to apply high-frequency waves to the patient's skin under a condition of the inductance of the impedance matching part 110 determined in the inductance determination step.

Before the reference impedance setting step S100, a preparation step may be further provided to position a patient's treatment target region between RF electrodes and generate high-frequency waves with the minimum power, which is less than power used in actual treatment and enables impedance matching operation.

In the reflected wave minimum value search step S400, while the inductance of the first variable inductor part 111 and the second variable inductor part 112 are varied, inflection points of measured reflected wave intensity are found. When a plurality of inflection points are found, a reflected wave minimum value having the lowest reflected wave intensity among the plurality of inflection points is found.

In the meantime, when high-frequency waves are applied and the medical procedure at the corresponding position is completed and it is intended to apply high-frequency waves to a new medical procedure position, the reference impedance setting step S100 and the minimum impedance setting step S200 are omitted, and the method is repeated starting from the reflected wave measurement step S300.

Herein, when it is intended to search for a reflected wave minimum value at a new medical procedure position, the inductance corresponding to the reflected wave minimum value at the previous medical procedure position and an inductance value adjacent to the reflected wave minimum value are preferentially used to search reflected waves.

A process of searching for a reflected wave minimum value and a reflected wave minimum value at a new medical procedure position will be described with reference to FIG. 4. First, the first reflected wave was measured (at an n-th time) using a preset initial inductance. Based on this, the inductance was changed and corresponding reflected waves were measured (an n+1-th time and an n+2-th time). The reflected wave at the n+2-th time was less than the initial reflected wave at the n-th time, so the inductance was changed again on the basis of the n+2-th time to measure a reflected wave (an n+3-th time). Herein, the greater reflected wave was measured again at the n+3-th time, so an inflection point was regarded as being between the n+3-th time and the n-th time. This was repeated to search for a reflected wave minimum value. In an embodiment of the present disclosure, it is assumed that the reflected wave measured at the n+2 th time was close to a minimum value.

After the medical procedure is completed by applying high-frequency waves, when moving from the medical procedure region to a new medical procedure region adjacent thereto takes place, the reflected waves and the inductance values at the previous medical procedure region are preferentially used to search for a reflected wave minimum value at the new medical procedure region. Since there is a high probability that an impedance difference between adjacent medical procedure regions is not large, an inductance combination under the condition in which the reflected wave minimum value at the previous position is obtained is preferentially used to search for a reflected wave minimum value. The inductance condition (the n+2-th time) in which the reflected wave minimum value was measured at the previous medical procedure position was used to measure reflected waves at the new medical procedure position (the n+2-th time → an m-th time). Afterward, an inductance condition close to the minimum value was used to measure reflected waves at the new medical procedure position (the n-th time → an m+1-th time), (the n+3-th time → an m+2-th time). When a reflected wave minimum value is not found during the process, the reflected wave minimum value is found by changing inductances.

Although the present disclosure is described with reference to specific items such as specific elements, to merely some embodiments, and to drawings, such specific details disclosed herein are merely representative for purposes of helping more comprehensive understanding of the present disclosure. The present disclosure, however, is not limited to only the exemplary embodiments set forth herein, and those skilled in the art will appreciate that the present disclosure can be embodied in many alternate forms.

Therefore, the spirit of the present disclosure shall not be limited to the above-described embodiments, and the entire scope of the appended claims and their equivalents will fall within the scope and spirit of the disclosure.

## Claims

1. A real-time impedance matching method for a high-frequency treatment device, the method comprising:
fixing, to a preset inductance in a reference impedance setting step, inductance of an impedance matching part for measuring impedance in the high-frequency treatment device and impedance in a patient's body;
applying, in a minimum impedance search step, a search voltage lower than a medical procedure voltage and searching for a minimum impedance by changing the inductance of the impedance matching part;
measuring, in a reflected wave measurement step, at least two reflected waves under a condition in which the inductance of the impedance matching part is varied;
comparing, in a reflected wave minimum value search step, the measured reflected waves to search for a reflected wave minimum value;
determining, in an inductance determination step, the inductance of the impedance matching part that satisfies the reflected wave minimum value; and
operating, in a high-frequency application step, the high-frequency treatment device with the medical procedure voltage to apply high-frequency waves to the patient's skin under a condition of the inductance of the impedance matching part determined in the inductance determination step.

2. The method of claim 1, wherein after the high-frequency application step, the steps starting from the reflected wave measurement step are repeated when it is intended to apply high-frequency waves to a new medical procedure position on the patient's skin.

3. The method of claim 2, wherein in the reflected wave minimum value search step, while the inductance of the impedance matching part is varied, a plurality of inflection points of measured reflected wave intensity are found, and the reflected wave minimum value having a lowest reflected wave intensity among the plurality of inflection points is found.

4. The method of claim 3, wherein when it is intended to search for a reflected wave minimum value at the new medical procedure position, reflected waves at the new medical procedure position are found preferentially using inductance corresponding to a reflected wave minimum value at a previous medical procedure position and an inductance value adjacent to the reflected wave minimum value.

5. The method of claim 1, wherein the impedance matching part comprises:
a first variable inductor part configured to measure the impedance in the high-frequency treatment device; and
a second variable inductor part configured to measure the impedance in the patient's body,
wherein inductance of the first variable inductor part and inductance of the second variable inductor part are the same.

6. The method of claim 5, wherein each of the first variable inductor part and the first variable inductor part comprises at least two unit inductors connected in series, and each of the at least two unit inductors comprises an inductor and a switch connected in parallel.

7. The method of claim 6, wherein a plurality of the inductors constituting the unit inductors have different proportionality constants.
